Europäisches Patentamt

(19) European Patent Office

Office européen des brevets

(11) EP 0 730 872 A1

(12) EUROPEAN PATENT APPLICATION

(43) Date of publication:
11.09.1996 Bulletin 1996/37

(51) Int. Cl.$^6$: **A61K 51/12**

(21) Application number: 95401505.3

(22) Date of filing: 23.06.1995

(84) Designated Contracting States:
AT BE CH DE DK ES FR GB GR IE IT LI LU MC NL PT SE

(30) Priority: 07.03.1995 KR 9504642

(71) Applicant: KOREA ATOMIC ENERGY RESEARCH INSTITUTE
Daejeon-si (KR)

(72) Inventors:
• Park, Kyoung-Bae
  Seoul (KR)
• Kim, Jae-Rock
  Sungdong-ku, Seoul (KR)
• Lee, Jong-Du
  Yangchun-ku, Seoul (KR)

(74) Representative: Hirsch, Marc-Roger et al
Cabinet Hirsch
34 rue de Bassano
75008 Paris (FR)

(54) **Radioactive patch for curing a skin disease and manufacturing method thereof**

(57) The invention relates to a radioctive patch of $^{165}$Dy, $^{166}$Ho, $^{90}$Y, $^{32}$P, $^{192}$Ir a combination thereof, said patch being obtained by neutron irradiating a pre-made sealed patch containing non-radioactive $^{164}$Dy, $^{165}$Ho, $^{89}$Y, $^{31}$P, $^{191}$Ir or a combination thereof.

The irradiated patch thereby emits beta or gamma rays.

The invention may be applied to the curing of skin disease.

FIG. 1

## Description

### BACKGROUND OF THE INVENTION

The present invention relates to a radioactive patch for curing skin disease and manufacturing method thereof.

Particularly flat epidermis cancer among skin diseases is a disease much generating when excessively exposed to sunlight for prime age or old age class, and other than that, it can be induced also by an environmental pollution material, carcinogenic substance, chronic skin ulcer, drainage portion in chronic osteomyelitis, a burn, and radiation exposure and the like. Although the generating rate is much different in response to human race, national and individual difference, generally it is many in white race, and in case of Canada, a skin cancer generating rate of 39 persons per 100,000 persons is reported.

Treatment of skin cancer would be various, and it is roughly classified into a surgical operation and a radiation medical cure. In case when a tumor is present at one region, a surgical operation would be carried out and good curing effect would be obtained, however since occasionally the skin cancer can be revealed to occur in many places, a skin graft is not easy after surgery, and there is many of inconvenient so as to do an anesthesia and enter a hospital.

A radiation cure becomes to do in case when a surgery is difficult, rind in this case the radiation has to be irradiated in a manner that the radiation of 5,000 rad should be irradiated five(5) times per one week for two weeks shortly, and generally for six weeks by dividing. Its curing effect is good but its shortcoming is that a radiation damage of neighboring organ especially bone or cartilage is worried because a permeability of radiation (X-ray) is strong.

In order to correct and complete an advantage and disadvantage of this each curing method, the present invention is originally invented with' creative idea. A patch in which fine particles of nuclear species ($^{165}$Dy, $^{166}$Ho, $^{32}$P, $^{90}$Y) emitting beta-rays or nuclear species ($^{192}$Ir) emitting gamma-rays of strong energy (1.4-2.2 MeV) are mixed with adhesive agent and coated on thin film and then laminated with polyethylene film or directly applying on the polyethylene film whereby making a sealed material of thin plate shape, can be appropriately adjusted in response to a pattern and magnitude of an affected part, and a radioactivity is increased in proportion to a particle quantity and an area of patch.

A curing method in accordance with the present invention can simply cure by irradiating beta-rays of 5,000-10,000 rad only one time for 30-60 minutes by directly adhering a radioactive patch to an affected part of skin diseases. And, it is very convenient since a medical treatment can be also concurrently done by same method to many place occurring diseases. In contrast to surgical operation, various problems of an anesthesia, an entering hospital, a pain and a skin grafting can be excluded, and in comparison with an existing radiation (X-ray) curing method as well, since the curing period is very short (every time 30-60 minutes for 5 weeks) and an attending hospital period can be greatly reduced, it has a great advantage. Since the patch of the present invention emits beta-rays, radiation damage can almost be prevented by blocking with this aluminum plate and the like for a normal region which is not directly contacting to surface thereof, therefore there is no adverse effect such as a suffering from irradiation of internal organs or breakage of cartilage or bones which have been worried in the existing radiation curing, and there is also advantage that an effected part of large region being difficult to operate can be treated.

A skin cancer of magnitude about 3-5 mm in diameter was induced to ten(10) white mouses by a carcinogenic substance and then a holmium-166 (Ho-166) patch (diameter 0.5cm) of 0.6 m Ci was adhered to an effected part for four(4) hours and an amount of 7,000-8,000 rad was irradiated. At this moment, since about 45% was irradiated as a result of measuring an absorbed dose amount of actual tumor by TLD, the dose amount accumulated to practical tumor was about 3,500 rad. The tumor was disappeared one week after irradiation and a dermatitis by radiation is generated in part at around of neighbor, but the dermatitis was all naturally cured after seven(7) weeks and it was confirmed that the tumor on tissue test was completely disappeared.

### Brief Description of the Attached Figures

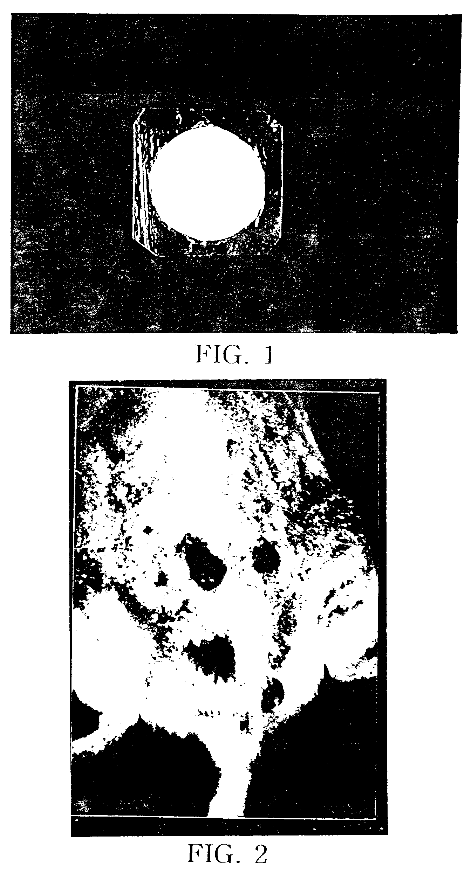

Fig. 1 is a radioactive $^{166}$HO-patch;
Fig. 2 is a view of generated skin cancer on white mouse before curing;

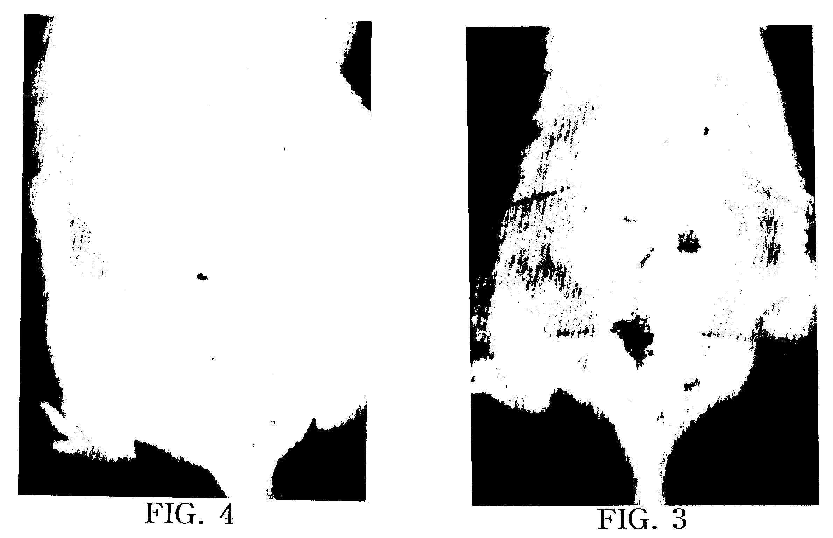

Fig. 3 is a view of adhered radioactive $^{166}$HO-patch around of skin cancer;
Fig. 4 is a view of disappearance of the skin cancer one week after curing.

Details of the present invention will be described in following examples.

### EXAMPLE 1

#### Preparation of holmium metallic macroaggregates($^{165}$Ho-MA)

60 mg of Ho(NO$_3$)$_3$.5H$_2$O or HoCl$_3$ is dissolved in 0.4 ml of distilled water and then 200 mg of sodium borohydride and 2 ml of 0.2N NaOH are added. Hydrogen is intensely generated and a precipitation is simultaneously generated This precipitation is ultrasonicator formed for two(2) minutes and then treated with separated and washed five(5) times with 5 ml of distilled water. After washing two(2) times with 5 ml of acetone, dried at room temperature.

EXAMPLE 2

Preparation of dysprosium metallic macroaggregates ($^{164}$Dy-MA)

This macroaggregates are prepared from $Dy(NO_3)_3$ or $DyCl_3$ compound by the same chemical processing as shown in example 1.

EXAMPLE 3

Preparation of iridium metallic macroaggregates ($^{191}$Ir-MA)

This macroaggregates are prepared from $IrCl_4$ or Ir(IV) compound by the same chemical processing as shown in example 1.

EXAMPLE 4

Preparation of $^{164}$Dy-MA and $^{166}$Ho-MA patch

$^{164}$Dy-MA or $^{165}$Ho-MA (1-5 µm in diameter) is coated on thin paper and then a circular patch of 0.05-5 cm in diameter was prepared. This patch is laminated with polyethylene film and sealed so that said particle is not leaked to outside of film. And, it was directly prepared by directly applying to the polyethylene film by same method.

EXAMPLE 5

Preparation of Yttrium ($^{89}$Y) and phosphorous ($^{31}$P) patch

These patch was prepared from $Y_2O_3$ or red phosphorous powder by the same processing method as shown in example 3.

EXAMPLE 6

Preparation of radioactive $^{165}$Dy, $^{166}$Ho, $^{32}$P, $^{90}$Y, $^{192}$Ir patch

The patch of above $^{164}$Dy, $^{165}$Ho, $^{31}$P, $^{89}$Y, $^{191}$Ir respectively as target material were irradiated in nuclear reactor and nuclear reaction (n,γ) was carried out and a radioactive patch corresponding to each was prepared.

EXAMPLE 7

Safety examination of radioactive patch

Respective radioactive patch was put into beaker contained with water and intensely stirred by a magnetic stirrer and simultaneously the radioactivity within the water was measured by a γ-counter at every time interval. A particle or radioactivity leaking out to exterior of the patch was not entirely detected.

EXAMPLE 8

Inducement of skin cancer on white mouse

15 µmol of 12-0-tetradecanoyl-13-acetate to 100 white shaved mice and then applied with about 0.1 ml and 2 µmol of [2'-(4-nitrophnoxy)] oxirans dissolved in acetone and applied two(2) times by one week. When a tumor would be generated by applying by this method for thirty-five (35) weeks, waited until to become a predetermined magnitude and thereafter confirming a generation of flat epidermis cancer and then met to cure.

EXAMPLE 9

A curing method utilizing a radioactive patch

A circular patch of a magnitude of about 1 cm in diameter was closely adhered to an effected part and a time was so determined that about 5,000 rad was irradiated to tumor region. An absorbed quantity was calculated by following expression.

In case of holmium-166 patch,

$$A = A_D \int_0^t e^{-0.693t/27} dt$$

Δ :        Equilibrium absorbed dose constant

$$\Sigma\Delta i = 3.486 \text{ g} \cdot \text{rad} / \mu\text{Ci} \cdot \text{hr}$$

Total energy absorbed dose :

$$A\Sigma\phi i (\gamma_k - \gamma_h) \Delta i$$

Wherein $\phi i = (\beta \text{ particle})$, $\gamma_k - \gamma_h - 1$

**Claims**

1. Radioactive patch of $^{165}$Dy, $^{166}$Ho, $^{90}$Y, $^{32}$P, or a combination thereof, said patch emitting beta-rays and being obtained by neutron irradiating a pre-made sealed patch containing non-radioactive $^{164}$Dy, $^{165}$Ho, $^{89}$Y, $^{31}$P, or a combination thereof.

2. Radioactive patch according to claim 1, characterized in that said pre-made sealed patch is prepared by applying on a support non-radioactive metallic particles $^{164}$Dy, $^{165}$Ho, $^{89}$Y, $^{31}$P oxides, or a combination thereof.

3. Radioactive patch according to claim 2, characterized in that said non-radioactive metallic particles have a diameter between 1 and 5 µm.

4. Radioactive patch according to claim 2 or 3, characterized in that said support is paper or polyethylene film.

5. Radioactive patch of $^{192}$Ir, said patch emitting gamma-rays and being obtained by neutron irradiating a pre-made sealed patch containing non-radioactive $^{191}$Ir.

6. Radioactive patch according to claim 5, characterized in that said pre-made sealed patch is prepared by applying on a support non-radioactive metal particles of $^{191}$Ir oxides.

7. Radioactive patch according to claim 6, characterized in that said non-radioactive metal particles have a diameter between 1 and 5 $\mu$m.

8. Radioactive patch according to claim 6 or 7, characterized in that said support is paper or polyethylene film.

9. Method for manufacturing a radioactive patch for skin disease, comprising applying dysprosium metallic particles ($^{164}$Dy-MA), holmium metallic particles ($^{165}$Ho-MA), $Y_2O_3$ or red phosphorous powder, or a combination thereof on a support, preparing a patch, neutron irradiating said patch, whereby said patch is nuclear reacted in a (n,$\gamma$) reaction to obtain a radioactive patch of $^{165}$Dy, $^{166}$Ho, $^{90}$Y, $^{32}$P, or a combination thereof.

10. Method for manufacturing a radioactive patch according to claim 9, characterized in that said patch is circular with a diameter between 0,05 and 5 cm.

11. Method for manufacturing a radioactive patch according to claim 9 or 10, characterized in that said patch is neutron irradiated in a nuclear reactor.

FIG. 1

FIG. 2

FIG. 3

FIG. 4

European Patent
Office

**EUROPEAN SEARCH REPORT**

Application Number

EP 95 40 1505

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | CHEMICAL ABSTRACTS, vol. 123, no. 8, 21 August 1995 Columbus, Ohio, US; abstract no. 93167, KAWASHITA, M. ET AL: "Preparation of glasses for radiotherapy by ion implantation" XP002007383 * abstract * & RADIAT. PHYS. CHEM. (1995), 46(2), 269-74 CODEN: RPCHDM;ISSN: 0146-5724, 1995, | 1-11 | A61K51/12 |
| Y | CHEMICAL ABSTRACTS, vol. 123, no. 21, 20 November 1995 Columbus, Ohio, US; abstract no. 279925, YAO, T. ET AL: "Application of ion implantation in a glass for radiotherapy" XP002007384 * abstract * & TRANS. MATER. RES. SOC. JPN. (1994), 17(LASER AND ION BEAM MODIFICATION OF MATERIALS), 507-10 CODEN: TMRJE3, 1994, | 1-11 | |
| Y | US-A-4 946 435 (SUTHANTHIRAN KRISHNAN ET AL) 7 August 1990 * column 2, line 61 - column 3, line 4; claims * | 1-11 | TECHNICAL FIELDS SEARCHED (Int.Cl.6)  A61K |

The present search report has been drawn up for all claims

| Place of search | Date of completion of the search | Examiner |
|---|---|---|
| THE HAGUE | 3 July 1996 | Berte, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)